Europäisches Patentamt

(19) European Patent Office

Office européen des brevets

(11) Publication number: **0 162 554**
A1

# (12) EUROPEAN PATENT APPLICATION

(21) Application number: 85302278.8

(22) Date of filing: 02.04.85

(51) Int. Cl.⁴: **B 01 J 32/00**
B 01 J 23/00, B 01 J 27/00
C 07 C 1/04

(30) Priority: 05.04.84 GB 8408803
19.09.84 GB 8423742

(43) Date of publication of application:
27.11.85 Bulletin 85/48

(84) Designated Contracting States:
BE DE FR GB IT NL

(71) Applicant: The British Petroleum Company p.l.c.
Britannic House Moor Lane
London EC2Y 9BU(GB)

(72) Inventor: Atkins, Martin Philip
The Britisch Petroleum Com. p.l.c. Chertsey Road
Sunbury-on-Thames Middlesex, TW16 7NL(GB)

(72) Inventor: Nay, Barry
The Britisch Petroleum Com. p.l.c. Chertsey Road
Sunbury-on-Thames Middlesex, TW16 7NL(GB)

(74) Representative: Harry, John et al,
BP INTERNATIONAL LIMITED Patents Division Chertsey
Road
Sunbury-on-Thames Middlesex TW16 7LN(GB)

(54) Catalyst, process for its preparation and use thereof in conversion of synthesis gas to hydrocarbons.

(57) A composition suitable for use after activation as a catalyst or a support therefor in the conversion of synthesis gas to hydrocarbons comprising a porous, essentially amorphous framework matrix comprising at least one element present in the form of a hydrolysed compound thereof, for example silicon and aluminium, and distributed uniformly throughout the framework matrix at least one metal selected from Groups VIa and VIII of the Periodic Table, for example iron, cobalt, nickel and/or ruthenium. Also a process for the production of the composition involving a hydrolysis step and a process for converting synthesis gas to hydrocarbons using the activated composition as catalyst.

EP 0 162 554 A1

1

## CATALYST PROCESS FOR ITS PREPARATION AND USE THEREOF
## IN CONVERSION OF SYNTHESIS GAS TO HYDROCARBONS

The present invention relates to catalysts and catalyst supports suitable for use in the conversion of synthesis gas to hydrocarbons, a process for their preparation and uses thereof.

During the 1970's, events in the petroleum industry stimulated research into alternatives to naphtha as the raw materials source for petrochemicals production. Interest was resurrected worldwide in process routes involving the use of $C_1$ feedstocks such as carbon monoxide which is potentially available on a vast scale from a variety of sources, including coal and natural gas. Continued development of coal gasification, hydrocarbon reforming and partial oxidation technologies, will increase the availability and should lower the unit cost of synthesis gas, which principally comprises carbon monoxide and hydrogen. The technology is also available for converting methane to synthesis gas. A process whereby carbon monoxide is catalytically hydrogenated to produce olefins and linear paraffins, generally referred to as the Fischer-Tropsch process, much worked on in the 1930's and 40's has recently been extensively re-examined.

The methods by which supported catalysts are generally prepared fall into three main general groups or modifications thereof as follows:-

(i) Mixing the support and active components by mechanical means, such as grinding, kneading or the like, followed by shaping and activating by thermal or chemical treatment,

1

(ii) impregnating a shaped or unshaped support with aqueous or non-aqueous solutions of readily decomposable salts or complexes of the active components followed by thermal decomposition and/or chemical activation thereof,

(iii) chemical precipitation of the active components together with or in the presence of unshaped supports.

Deficiencies have been recognised in all these methods, see for example GB-A-1342020 which recognises the problem of catalyst deactivation.

Catalysts for the Fischer-Tropsch process are generally prepared by method (ii), i.e. by impregnating or ion-exchanging a Group VIII metal component, for example salts of ruthenium and/or iron, together with a promoter ion (typically an alkali metal ion, for example $Na^+$ or $K^+$) on to a support such as alumina or silicalite. A perpetual problem associated with the use of Fischer-Tropsch type catalysts has been their lack of selectivity to liquid aliphatic hydrocarbons as compared with carbon dioxide or methane for example.

In EP-A-72 612 [Coal Industry (Patents) Limited] there is described a synthesis gas conversion catalyst which can be used in the direct conversion of synthesis gas to olefinic hydrocarbons in high yield. The catalyst comprises a highly porous amorphous silica support, wherein the support has a monolayer of silica deposited on it and is impregnated with a transition metal, the catalyst having a maximum pore diameter of up to 5nm and an average pore diameter of up to 1.5nm. Such a catalyst can be produced by treating a support of a highly porous amorphous silica with a solution of a hydrolysable compound of silicon, removing the solvent to leave a monolayer of the compound on the surface area of the support, hydrolysing the compound to produce a monolayer of silica on the support and to produce a catalyst having a maximum pore diameter of up to 5nm and an average pore diameter of up to 1.5nm, and impregnating the catalyst with a transition metal.

In the prior art catalyst compositions the catalytically active metal components are generally either present in the pores of the support or dispersed on its surface in a non-uniform distribution.

The present invention provides a composition suitable for use after activation as a catalyst or a catalyst support in the conversion of synthesis gas to hydrocarbons which composition comprises a porous, essentially amorphous framework matrix comprising at least one element present in the form of a hydrolysed compound thereof, and at least one metal selected from Groups VIa and VIII of the Periodic Table of the Elements in compound form, the metal(s) being distributed uniformly throughout the framework matrix.

The compositions according to the invention are essentially monolithic, i.e. they constitute one massive undifferentiated whole.

The Periodic Table as used throughout the present specification is that found in Advanced Inorganic Chemistry (2nd Edn.) by F.A. Cotton and G. Wilkinson, Interscience, 1966.

In another aspect the invention provides a process for the production of a composition as hereinbefore described which process comprises hydrolysing a homogeneous mixture comprising (i) at least one element having a hydrolysable compound in the form of a hydrolysable compound thereof, (ii) a hydrolysis medium and (iii) at least one metal selected from Groups VIa and VIII of the Periodic Table of the Elements in the form of a compound soluble under hydrolysis conditions in the hydrolysis medium, and thereafter removing the hydrolysis medium and hydrolysate moiety not containing the matrix element(s).

Suitable elements having a hydrolysable compound include elements of Groups IIa, IVa, Va, IIIb, IVb of the Periodic Table and the rare earth elements. Examples of suitable elements include silicon, aluminium, gallium, magnesium, calcium, phosphorus, titanium, beryllium, vanadium, lanthanum and cerium, of which silican and/or aluminium are preferred. Suitable hydrolysable compounds of silicon include the tetra-alkyl orthosilicates, for example tetraethyl orthosilicate. Suitable hydrolysable compounds of aluminium, titanium, calcium, magnesium and lanthanum, for example, include the metal alkoxides and metal carboxylates.

Provided that a hydrolysable compound of one of the aforesaid elements is present in the hydrolysis mixture, other of the elements may be present, if desired, in the form of soluble hydrolysable compounds or non-hydrolysable compounds. Thus, for example, silicon may be present in the mixture in the form of a hydrolysable compound and aluminium may be present in the mixture in the form of a non-hydrolysable or hydrolysable compound and vice-versa.

The metals of Groups VIa and VIII of the Periodic Table are chromium, molybdenum, tungsten, iron, cobalt, nickel, ruthenium, rhodium, palladium, osmium, iridium, and platinum, of which at least one of iron, cobalt, nickel and ruthenium is preferred. The metal(s) may suitably be added to the hydrolysis mixture in the form of a thermally decomposable and/or reducible salt(s) thereof soluble in the mixture, for example a halide, a nitrate or a sulphate.

The composition according to the invention preferably further includes a promoter comprising at least one alkali metal, alkaline earth metal or rare earth metal, of which the alkali metal is preferred. Preferred alkali metals are sodium and potassium. The further metal is preferably incorporated in the composition by addition, in the form of a soluble compound thereof, to the homogeneous mixture as component (iv) thereof.

The composition according to the invention preferably includes a halide component, preferably chloride, which may suitably be incorporated in the composition by providing one of the components of the homogeneous mixture, for example component (iii), as a halide salt. Alternatively, the composition may be halogenated in conventional manner.

The element(s) comprising the framework matrix may suitably be present in the composition in an amount greater than 50% w/w, preferably greater than 75% w/w. The metal(s) of Group VIa or Group VIII may suitably be present in an amount greater than 0.05% w/w, preferably greater than 0.1% w/w. The promoter may suitably be present in an amount up to 5% w/w. Halide may suitably be present in an amount up to 1% w/w.

As regards the process for producing compositions as aforesaid, component (ii) of the hydrolysis mixture is a hydrolysis medium, which may suitably be water, though other media optionally containing a hydrolysing agent, such as for example, ammoniacal alkanol, may be employed if desired.

It is essential for the purposes of the invention that the hydrolysis mixture be homogeneous, that is to say that the components be uniformly distributed throughout the mixture. Means for achieving homogeneity will be readily apparent to those skilled in the art. This may suitably be accomplished for example by stirring or shaking the mixture.

The order of addition of the components to the mixture must be such as to ensure that the mixture is homogeneous during the hydrolysis step. Certain combinations of components and orders of addition may combine to produce conditions, for example very rapid gelation, which prevent the effective homogenisation of subsequently added components. Thus, for example, in the preparation of a catalyst comprising ruthenium, iron and potassium distributed uniformly throughout a silica/alumina framework matrix the aluminium compound should be added after the potassium hydroxide otherwise the mixture is prone to rapid gelation.

The hydrolysis conditions to be employed will depend upon many factors, including the nature of the hydrolysable compound and the nature of the hydrolysis medium. Using tetra-alkyl orthosilicates as the hydrolysable compounds and water as the hydrolysis medium for example, the hydrolysis may suitably be carried out at a temperature in the range from 25 to 100°C for a period sufficient to effect substantially complete hydrolysis of the compound, for example from 1 to 12 hours, during which time homogeneity of the mixture should be maintained.

Thereafter the hydrolysis medium and the hydrolysate moiety not containing matrix element(s) is removed. Generally, by this point the homogeneous mixture will have gelled, if not it will probably do so as the hydrolysis medium is progressively removed. The hydrolysis medium and the hydrolysate moiety may be removed in any convenient manner, for example by evaporation. Using tetraalkyl orthosilicates as the hydrolysable compound for example, the hydrolysate moiety not containing matrix element(s) is an alkanol. It may be advantageous under certain circumstances to use subatmospheric pressures during the evaporation. The evaporation temperature is preferably maintained below 200°C.

Thereafter, the composition may suitably be heated at a temperature in the range from 100 to 600°C, preferably from 100 to 150°C, for a suitable period, for example greater than 6 hours for the purpose of dehydrating the composition.

In a preferred embodiment, the invention provides a composition suitable for use after activation in the conversion of synthesis gas to hydrocarbons which composition comprises a chloride component, a porous, essentially amorphous framework matrix comprising silicon and aluminium either or both of which are in the form of hydrolysed compounds thereof and the metals ruthenium, iron and potassium, the metals ruthenium, iron and potassium at least being distributed uniformly throughout the framework matrix.

The catalyst composition may suitably be produced by hydrolysing a homogeneous mixture comprising water, a hydrolysable silicon compound, a water soluble aluminium compound, a source of halide ions, a soluble ruthenium compound and a soluble potassium compound and thereafter removing the water and the hydrolysate moiety not containing silicon.

In the aforesaid composition, the amount of potassium may suitably be in the range from 0.05 to 10% w/w, preferably from 0.1 to 5% w/w; the amount of ruthenium and iron may suitably be in the range from 0.1 to 25% w/w, preferably from 0.5 to 15% w/w; the amount of halide may suitably be in the range from 0.05 to 5% w/w, preferably from 0.1 to 2.5% w/w, the remainder of the composition being comprised of silicon and aluminium. The silicon to aluminium ratio may suitably be greater than 1:1. It has been found that for use as a catalyst in the conversion of synthesis gas to hydrocarbons, the yield of liquid products in the gasoline boiling range ($C_5^+$ hydrocarbons) is very much dependent on the silicon to aluminium ratio of the catalyst. Thus, for this purpose the silicon to aluminium ratio may suitably be in the range from 1:1 to 50:1, preferably from 3:1 to 20:1, even more preferably from 5:1 to 15:1. Here and elsewhere in the specification, the silicon to aluminium ratio is to be understood as the ratio of the number of atoms of silicon to the number of atoms of aluminium.

Before use as a catalyst for the conversion of synthesis gas to hydrocarbons it is preferred to reductively activate the compositions of the present invention. Reductive activation may suitably be effected by heating the composition, suitably at a temperature in the range from 200 to 600°C, in a reducing atmosphere, for example hydrogen, carbon monoxide or synthesis gas. It is believed that the reductive activation treatment chemically reduces the catalytic metal compound to the active metallic state. Reductive activation may be effected in a separate, distinct step or "in situ" during the conversion of synthesis gas to hydrocarbons.

In another aspect the invention provides a process for the conversion of synthesis gas to hydrocarbons which process comprises contacting synthesis gas at elevated temperature and pressure with a catalytically effective amount of a composition as hereinbefore described.

A particularly suitable composition comprises iron, ruthenium and potassium uniformly distributed throughout an amorphous silica-alumina framework matrix, prepared and activated as described hereinbefore.

Preferably the composition is activated prior to use in the process of the invention.

Catalysts containing these essential components and prepared by the method of the invention can convert synthesis gas at lower temperatures to hydrocarbons with much higher selectivities to desirable hydrocarbons and lower selectivities to unwanted carbon dioxide than catalysts in which the catalytically active components are impregnated in similar proportions on to the support. Methods for preparing synthesis gas are well known in the art and usually involve the partial oxidation of a carbonaceous substance, e.g. coal. Alternatively, synthesis gas may be prepared, for example, by the catalytic steam reforming of methane. Although it is preferred to use substantially pure synthesis gas, the presence of such impurities as carbon dioxide and nitrogen can be tolerated. On the other hand, impurities which have a deleterious effect on the reaction should be avoided. The ratio of hydrogen to carbon monoxide in the synthesis gas may vary widely. Normally, the molar ratio of hydrogen to carbon monoxide may be in the range from 10:1 to 1:10, preferably from 5:1 to 1:5. Methods for adjusting the molar ratio of hydrogen to carbon monoxide by the so-called shift reaction are well known in the art.

The synthesis gas may suitably be contacted with the catalyst at an elevated temperature in the range 150 to 450°C, preferably from 225 to 375°C. The pressure may suitably be in the range from atmospheric to 100 bars.

The process may be operated batchwise or continuously, preferably continuously. The contact time in a continuous process, defined as:

$$\frac{\text{Volume of catalyst (in millilitres)}}{\text{Total volume of gas (in millilitres per second at NTP)}}$$

may suitably be in the range from 1 to 30 seconds, preferably from 1 to 10 seconds, though longer and shorter times may be used if desired.

The catalyst may be employed in any suitable form, for example in the form of either a fixed bed, a fluidised bed or a moving bed.

The invention will now be further described by reference to the following Examples.

A. Catalyst Preparation

Example 1

$Ru_{0.12}$ $Fe_{0.16}$ $K_{0.11}$ support $_{6.8}$ (silicon to aluminium ratio of 5:1)

Tetra-alkyl orthosilicate (tetraethoxy silane) (52.1g) was placed in a round bottom flask equipped with thermometer, condenser and suba seal. To the rapidly stirred material was added ruthenium chloride (1.68g), ferric nitrate (6.51g) and potassium hydroxide (1.08g) followed by aluminium nitrate (20.1g) in 146ml deionised water.

The suspension was heated to 70°C and stirring continued until hydrolysis of the tetraalkyl orthosilicate was complete. The material was then heated in an oven at 80 to 100°C for 12h to produce a brown glass-like solid.

The levels of ruthenium and iron in the material were 1.6 and 2.2% b.w. respectively and the silicon to aluminium ratio of the matrix was 5:1.

The catalyst so-obtained was reduced in a stream of hydrogen for 15 h at 225°C.

Examples 2 to 7

$Ru_{0.12}Fe_{0.16}K_{0.11}$ support $6.8$ (silicon to aluminium of X:1 wherein X = 1,3,7,10,20 and 50)

Example 1 was repeated except that the amounts of tetra-alkyl orthosilicate and aluminium nitrate were varied so as to provide catalyst compositions wherein the silicon to aluminium ratio was as follows:-

|       |   |           |
|-------|---|-----------|
| 1:1   | - | Example 2 |
| 3:1   | - | Example 3 |
| 7:1   | - | Example 4 |
| 10:1  | - | Example 5 |
| 20:1  | - | Example 6 |
| 50:1  | - | Example 7 |

Example 8

$Ru_{0.12}Fe_{0.16}K_{0.11}$ support $6.8$ (Si:Al of 10:1)

Example 5 was repeated except that instead of aluminium nitrate there was used aluminium isopropoxide.

Example 9

$Ru_{0.12}Fe_{0.16}K_{0.11}$ support $6.8$ (Si:Mg of 10:1)

Example 5 was repeated except that instead of aluminium nitrate there was used magnesium alkoxide.

Example 10

$Ru_{0.12}Fe_{0.16}K_{0.11}$ support $6.8$, $Cl^-$ (Si:Al = 10:1)

Example 5 was repeated. In this example the $[Cl^-]$ was determined; it was 1.0% w/w prior to reductive activation and 0.5% w/w after reductive activation.

Example 11

$Ru_{0.12}Fe_{0.16}$ support 6.8 (Si:Al = 10:1)

Example 5 was repeated except that the potassium hydroxide was omitted.

Example 12

$Fe_{0.16}$ support 6.8 (Si:Al = 10:1)

Example 5 was repeated except that both potassium hydroxide and ruthenium chloride were omitted.

Example 13

$Ru_{0.12}Fe_{0.16}$ support 6.8, no $Cl^-$ (Si:Al = 10:1)

Example 5 was repeated except that instead of ruthenium chloride there was used ruthenium acetylacetonate. The resulting catalyst did not in consequence contain chloride ion.

Example 14

$Fe_{0.16}K_{0.11}$ support 6.8 (Si:Al = 10:1)

Example 5 was repeated except that ruthenium chloride was omitted.

Example 15

$Co_{0.16}$ support 6.8 (Si:Al = 10:1)

Example 5 was repeated except that cobalt was added and ruthenium chloride, ferric nitrate and potassium hydroxide were omitted.

Comparison Test 1

The procedure of Example 1 was repeated except that the addition of ruthenium chloride, ferric nitrate and potassium hydroxide was omitted, i.e. an amorphous aluminosilicate having a silicon to aluminium ratio of 5:1 was synthesised. The support so-obtained was impregnated with ruthenium chloride, ferric nitrate and potassium hydroxide in the same ratio as in Example 1, to provide a catalyst of the composition $Ru_{0.12}$ $Fe_{0.16}$ $K_{0.11}$ support 6.8. The catalyst so obtained was reduced in a stream of hydrogen for 15h at 225°C.

10

B. Use of the catalysts for the conversion of synthesis gas

Examples 16-22

The catalysts obtained from Examples 1 to 7 were contacted in turn with a mixture of carbon monoxide and hydrogen (1:1 molar) at a temperature of 294°C, a pressure of 20 bar and a GHSV of 2330 (contact time 1.5s). The results obtained are shown in Table 1.

Comparison Test 2

The use of the catalyst obtained in Comparison Test 1 as a synthesis gas conversion catalyst was examined under comparable conditions (except that the temperature was 395°C) to those used in Example 16. The results are shown in Table 1.

## Table 1

| Example | Si:Al ratio | Catalyst | MOLAR SELECTIVITY (%) | | | |
|---|---|---|---|---|---|---|
| | | | $CH_4$ | $CO_2$ | Liquids* | Oxygenates |
| 17 | 1:1 | Example 2 | 33 | 8.5 | 30 | - |
| 18 | 3:1 | Example 3 | 28 | 6.0 | 31.5 | - |
| 16 | 5:1 | Example 1 | 20 | 5.0 | 36.0 | 4.2 |
| 19 | 7:1 | Example 4 | 18 | 6.0 | 51.5 | - |
| 20 | 10:1 | Example 5 | 16.5 | 4.9 | 61.5 | - |
| 21 | 20:1 | Example 6 | 22.6 | 15.3 | 28.4 | - |
| 22 | 50:1 | Example 7 | 36.0 | 15.6 | 15.7 | - |
| Comp Test 2 | 5:1 | Comp. Test 1 | 47.7 | 27.6 | trace | 0.3 |

* Liquids comprise $C_5$ and greater hydrocarbons.

- not measured

The results in the Table demonstrate the following advantages of a catalyst prepared according to Example 1 over a Fischer-Tropsch catalyst in which the support is prepared according to the method of Comparison Test 1 and in which the active components are impregnated on to the support in conventional fashion:-

(i)      considerably lower selectivity to carbon dioxide,

(ii)     considerably greater selectivity to liquid aliphatics,

(iii)    greater selectivity to oxygenates, and

(iv)     considerably lower reaction temperature for similar conversions of synthesis gas.

0162554

It can also be seen that the best yields of $C_5$ and greater liquid hydrocarbons occur in the Si:Al range of from 3:1 to 20:1 and more particularly in the range from 5:1 to 15:1 where the molar selectivities to unwanted methane and carbon dioxide are lowest.

Examples 23 and 24

The procedure of Examples 16 to 22 was repeated except that the GHSV was changed to 2504 and the catalysts of Examples 8 (Ex. 23) and 8 (Ex. 24) were used respectively instead of the catalysts as used in those Examples.

The results obtained are shown in Table 2.

Table 2

| Example | Catalyst | Temp. (°C) | CO conv. (%) | MOLAR SELECTIVITY (%) | | |
|---------|----------|------------|--------------|------|-----|-----|
|         |          |            |              | $CH_4$ | $CO_2$ | $C_3^+$ |
| 23      | Ex. 8    | 290        | 28.4         | 21.3 | 6.0 | 66.4 |
| 24      | Ex. 9    | 305        | 37.0         | 25.9 | 9.3 | 63.0 |

Examples 25 to 30

The catalysts obtained in Examples 10 to 15 were contacted in turn in a microreactor with a mixture of carbon monoxide and hydrogen (1:2 molar) at a pressure of 6 bar and a GHSV of 500. The products obtained at 225°C, 245°C and 265°C were analysed.

The results are given in Table 3.

0162554

Table 3

| Example | Catalyst | Temp (°C) | CO conv. (%) | MOLAR SELECTIVITY (%) | | |
|---|---|---|---|---|---|---|
| | | | | $CH_4$ | $CO_2$ | ORGANICS* |
| 25 | Ex. 10 | 225 | 21.0 | 20.0 | 4.0 | 76.0 |
| | | 245 | 60.0 | 23.0 | 3.0 | 74.0 |
| | | 265 | 60.0 | 35.0 | 3.0 | 62.0 |
| 26 | Ex. 11 | 225 | 12.4 | 20.7 | 11.3 | 88.7 |
| | | 245 | 10.7 | 45.9 | 9.7 | 90.3 |
| | | 265 | 28.7 | 33.9 | 4.7 | 95.3 |
| 27 | Ex. 12 | 225 | 49.5 | 60.6 | 12.1 | 87.9 |
| | | 245 | 2.2 | 77.9 | 22.1 | 77.9 |
| | | 265 | 1.3 | 80.3 | 19.7 | 80.3 |
| 28 | Ex. 13 | 225 | 6.3 | 38.6 | 34.1 | 65.9 |
| | | 245 | 17.0 | 23.6 | 22.1 | 77.8 |
| | | 265 | 32.4 | 25.6 | 20.6 | 79.4 |

* ORGANICS - hydrocarbon products including methane.

The results reported in Table 3 demonstrate:

(i)  the improved performance in terms of lower methane and $CO_2$ selectivities obtained with chloride present in the catalyst, cf Examples 25 and 28,

(ii)  the improved performance in terms of lower $CO_2$ selectivity associated with the presence of K in a Ru/Fe catalyst, cf Examples 25 and 26,

(iii) the improvement associated with the presence of Ru and K in an Fe catalyst, cf Examples 25, 27 and 29, and

(iv) the satisfactory performance of the Co catalyst in the absence of promoters, cf Example 30.

C.  Analytical

The composition of Example 1 was characterised prior to reductive activation by a variety of techniques including XRD, XRF, NMR and electron microscopy.

The material had a glass-like appearance.  The metal ions (Ru,Fe,K) and matrix components (Si,Al) are in a homogeneous distribution throughout the material as judged by particle morphology observed using scanning electron microscopy and by EDAX which reveals substantially no difference between the concentration

of metal ion/matrix components at different sampling points throughout the material.

The material is amorphous as judged by XRD powder patterns which show that no long range order is present in the material.

NMR analysis of the silica/alumina matrix material ($^{27}$Al and $^{29}$Si) show spectra characteristic of amorphous material. The aluminium appears to be present predominantly in octahedral coordination provided that the temperature is below about 150°C.

Infrared spectroscopy clearly shows nitrate ligand and a very broad hydroxyl peak showing the material to be substantially hydrated.

Surface Area and Pore Volume Distributions

The surface area and pore volumes of the compositions of Examples 3 to 5 were measured by a BET method. The results are given in Table 4.

Table 4

| Ex | BET Surface Area ($m^2g^{-1}$) | Pore Volume ($cm^3g^{-1}$) | Pore Volume (% pores below 20 Angstrom) | Si:Al Ratio |
|----|----|----|----|----|
| 3 | 285 | 0.143 | 67.8 | 3:1 |
| 4 | 371 | 0.185 | 78.3 | 7:1 |
| 5 | 494 | 0.234 | 90.9 | 10:1 |

With reference to Table 4, as the Si:Al ratio changes from 3:1 to 10:1 the surface area increases from 285 to $444m^2g^{-1}$ and the pore volume from 0.143 to 0.243 mlg$^{-1}$. In addition, the proportion of pores below 20 Angstrom rises from 67.8 to 90.9% as the Si:Al ratio increases from 3:1 to 10:1.

Acidity Measurements

The material in powder form (0.5g, 500 micromesh) was suspended in n-heptane (10ml). Indicator (dicinnamalacetone, pka = -3.0, or dimethyl yellow, pka = +3.3, 2 to 3 mg) was added to the stirred solution and left to equilibrate for 30 minutes. n-Butylamine (0.1 molar) in n-heptane was then added dropwise until the characteristic colour change of the indicator was observed.

The results are given in Table 5.

Table 5

| Ex | Si:Al ratio | n-butylamine titre (ml) | |
|----|-------------|---------------------------------|-------------------------------|
|    |             | Dimethyl yellow pKa = + 3.3 | Dicinnamalacetone pKa = − 3.0 |
| 3  | 3:1         | 5.1                             | 3.0                           |
| 1  | 5:1         | 4.0                             | 2.6                           |
| 4  | 7:1         | 4.4                             | 2.8                           |
| 5  | 10:1        | 5.9                             | 4.8                           |

With reference to Table 5, as the Si:Al ratio increases from 3:1 to 7:1, the acidity passes through a minimum but increases rapidly at 10:1.

Claims:

1. A composition suitable for use after activation as a catalyst or a catalyst support in the conversion of synthesis gas to hydrocarbons which composition comprises a porous, essentially amorphous framework matrix comprising at least one element present in the form of a hydrolysed compound thereof and at least one metal selected from Groups VIa and VIII of the Periodic Table of the Elements in compound form, the metal(s) being distributed uniformly throughout the framework matrix.

2. A composition according to claim 1 wherein the element having a hydrolysable compound is either silicon, aluminium, gallium, magnesium, calcium, phosphorus, titanium, beryllium, vanadium, lanthanum or cerium.

3. A composition according to claim 2 wherein the element is silicon and/or aluminium.

4. A composition according to any one of the preceding claims wherein the metal distributed uniformly throughout the framework matrix is one or more of iron, cobalt, nickel and ruthenium.

5. A composition according to any one of the preceding claims further including a promoter comprising at least one alkali metal, alkaline earth metal or rare earth metal.

6. A composition according to any one of the preceding claims further including a halide component.

7.   A composition suitable for use after activation in the conversion of synthesis gas to hydrocarbons which composition comprises a chloride component, the metals ruthenium, iron and potassium and a porous, essentially amorphous framework matrix comprising silicon and aluminium, either or both of which being in the form of a hydrolysed compound thereof, the metals ruthenium, iron and potassium at least being distributed uniformly throughout the framework matrix.

8.   A process for the production of a composition as claimed in claims 1 to 7 which process comprises hydrolysing a homogeneous mixture comprising (i) at least one element having a hydrolysable compound in the form of a hydrolysable compound thereof, (ii) a hydrolysis medium, and (iii) at least one metal selected from Groups VIa and VIII of the Periodic Table of the Elements in the form of a compound soluble under hydrolysis conditions in the hydrolysis medium, and thereafter removing the hydrolysis medium and hydrolysate moiety not containing the matrix element(s).

9.   A process according to claim 8 wherein a homogeneous mixture comprising water, a hydrolysable silicon compound, a water soluble aluminium compound, a source of chloride ions, a soluble ruthenium compound and a soluble potassium compound is hydrolysed and thereafter water and the hydrolysate moiety not containing silicon is removed.

10.   A process according to either claim 8 or claim 9 wherein the composition is thereafter heated at a temperature in the range from 100 to 600°C.

11.   A process according to any one of claims 8 to 10 wherein the composition is reductively activated by heating at a temperature in the range from 200 to 600°C in a reducing atmosphere.

12.   A process for the conversion of synthesis gas to hydrocarbons which process comprises contacting synthesis gas at elevated temperature and pressure with a catalytically effective amount of a reductively activated composition as claimed in claims 1 to 7.

13.   A process according to claim 12 wherein the composition of claim 7 is employed.

14. A process according to claim 13 wherein the silicon to aluminium ratio of the framework matrix is in the range from 3:1 to 20:1.

15. A process according to claim 14 wherein the silicon to aluminium ratio is in the range from 5:1 to 15:1.

European Patent
Office

EUROPEAN SEARCH REPORT

0162554

Application number

EP 85 30 2278

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-3 243 368 (B.F. MULASKEY)<br><br>* Column 3, lines 17-75; column 4, lines 1-41; column 11, lines 60-75 *<br><br>--- | 1-4,6, 8,10, 11,14, 15 | B 01 J 23/89<br>C 07 C 1/04<br>B 01 J 32/00<br>B 01 J 27/00 |
| A | FR-A-1 553 174 (THE BRITISH PETROLEUM CO.)<br><br>* Page 1, right-hand column, paragraph 2; page 2, right-hand column, paragraphs 3,4; page 4, example 3 *<br><br>--- | 1-4,6, 8,10, 14,15 | |
| A | FR-A-2 334 415 (CO. FRANCAISE DE RAFFINAGE)<br><br>* Page 5, lines 14-34; page 6, lines 9-11 *<br><br>--- | 1-4,6, 8,10, 14,15 | TECHNICAL FIELDS SEARCHED (Int. Cl.4)<br><br>B 01 J<br>C 07 C |
| A | NL-A-7 708 307 (SHELL)<br>* Page 9, lines 1-4; page 4, lines 1-10; page 6, lines 21-32 *<br><br>--- | 4-6,12 | |
| A | EP-A-0 071 770 (SCHERING AG)<br>* Page 3, lines 20-30; page 4, lines 28-29; page 6, lines 1-12 *<br><br>----- | 1,2,12 | |

The present search report has been drawn up for all claims

| Place of search | Date of completion of the search | Examiner |
|---|---|---|
| THE HAGUE | 30-07-1985 | KERRES F.M.G. |